# EUROPEAN PATENT APPLICATION

(11) **EP 4 733 394 A1**
(43) Date of publication of application: **29.04.2026**
(21) Application number: 25158809.1
(22) Date of filing: 19.02.2025
(51) Int. Cl.: C12N 9/12, C12Q 1/6844

(54) **REVERSE TRANSCRIPTASE ENZYMES, COMBINATIONS, METHODS AND USES THEREOF**

(30) Priority: 25.10.2024 PT 2024119792
(71) Applicant: Nzytech Lda, 1649-038 Lisboa (PT)
(72) Inventor: FOGAÇA DA FONSECA COSTA, INÊS MARIA, 1649-038 LISBOA (PT); PEDRO FERNANDES, VÂNIA ONDINA, 1649-038 LISBOA (PT); MENDES GODINHO DE ANDRADE FONTES, CARLOS, 1649-038 LISBOA (PT)
(74) Representative: Patentree

(57) **Abstract**

The present disclosure relates to novel reverse transcriptase enzymes, RTV18, RTV18FL, and combinations offering enhanced performance across a wider temperature range, faster reaction times, and increased resilience to inhibitors, thus improving the stability and the catalytic activity of reverse transcription, enabling a more efficient and reliable molecular biology diagnostic.

## Description

### TECHNICAL FIELD

The present disclosure relates to novel reverse transcriptase enzymes, combinations, methods and uses thereof for the field of molecular biology and biotechnology where it is required rapid and sensitive RNA detection.

The present invention also discloses a composition for use in reverse transcription as part of reverse transcription-polymerase chain reaction (RT-PCR) or reverse transcription loop-mediated isothermal amplification (RT-LAMP) reaction, comprising the enzyme RTV18 and/or or RTV18FL. The composition of the present disclosure applies to both diagnostic and research-based applications, improving the efficiency, accuracy, and speed of nucleic acid amplification.

### BACKGROUND

Reverse transcriptases (RTs) are enzymes that catalyse the synthesis of deoxyribonucleic acid (DNA) from a ribonucleic acid (RNA) template, a process known as reverse transcription. This process is central to the life cycle of retroviruses and also plays a role in key cellular processes, such as the maintenance of telomeres, which is coordinated by a specialized class of RTs known as telomerases. RTs were first discovered in retroviruses, where they function by converting the viral RNA genome into complementary DNA (cDNA). This cDNA is then integrated into the host genome and subsequently transcribed into viral RNA, allowing for the production of new viral proteins and progeny. The most well-characterized RTs are derived from retroviruses, such as the Moloney Murine Leukaemia Virus (M-MULV) reverse transcriptase, which serves as a model enzyme for studying the structure and function of RTs. RTs possess three major functional activities:
- RNA-dependent DNA polymerase activity: The primary enzymatic function, in which the RT catalyses the synthesis of a DNA strand using an RNA template. This is critical for converting RNA into DNA during reverse transcription.
- RNase H activity: RTs often include RNase H activity, which degrades the RNA strand in RNA-DNA hybrids, leaving a single-stranded DNA molecule that can be used as a template for further DNA synthesis.
- DNA-dependent DNA polymerase activity: In addition to synthesizing DNA from RNA, RTs can also extend DNA from a DNA template. While this activity is typically less efficient than RNA-dependent DNA synthesis, it remains a significant feature of some RT enzymes.

Beyond their biological roles, RTs are extensively used in molecular biology and diagnostics for various critical applications. These include cDNA synthesis, reverse transcription-polymerase chain reaction (RT-PCR), reverse transcription loop-mediated isothermal amplification (RT-LAMP) and RNA sequencing. These techniques are fundamental in both research and clinical diagnostics, enabling the detection and analysis of RNA sequences. To meet the demands of specific molecular applications, engineered RT variants have been developed with enhanced properties such as improved thermostability, increased processivity, and higher template specificity. These modified enzymes offer better performance in high-temperature assays, long-read cDNA synthesis, and other specialized workflows.

While some RTs, such as M-MULV RT, are well-characterized, many remain unexplored across different species. However, traditional reverse transcriptases, including M-MULV RT and its mutant derivatives, have several limitations. These include narrow temperature optima, slower enzymatic kinetics, and susceptibility to PCR inhibitors, all of which can reduce the overall efficiency and reliability of molecular workflows. Additionally, M-MULV requires longer reaction times (ca. 30 min). These limitations create a demand for more robust reverse transcriptases capable of maintaining high performance under a broader range of conditions and having a reduced reaction time.

The present disclosure solves the lack of reverse transcriptase enzymes with superior catalytic efficiency, faster reaction times, and robust stability under a wide range of environmental and reaction conditions.

These facts are disclosed in order to illustrate the technical problem addressed by the present disclosure.

### GENERAL DESCRIPTION

The present disclosure relates to novel reverse transcriptase enzymes, RTV18, RTV18FL, and combinations offering enhanced performance across a wider temperature range, faster reaction kinetics, and increased resistance to inhibitors. These improvements enhance the stability and the catalytic efficiency of reverse transcription, enabling more reliable and efficient use in molecular biology applications, including molecular diagnostics.

Surprisingly, the present disclosure can efficiently synthesize cDNA in as little as 1 minute, which represents a major improvement over traditional reverse transcriptases, such as M-MULV RT, which require much longer reaction times and lower reaction temperatures. The rapid kinetics of RTV18, RTV18FL, and combinations make these novel enzymes and blends a highly versatile tool for various applications where fast reaction times are essential, such as quantitative reverse transcription polymerase chain reaction (RT-qPCR), reverse transcription loop-mediated isothermal amplification (RT-LAMP), and other time-sensitive molecular biology protocols.

In an aspect of the present disclosure, RTV18, RTV18FL, and combinations have the ability to maintain high efficiency and sensitivity across a broad range of RNA inputs, temperatures and reaction times, which reinforces their superiority as next-generation reverse transcriptases for research and diagnostics. Moreover, the use of optimized storage buffers that maintain enzyme stability under diverse conditions enhances the applicability of RTV18, RTV18FL, and their combinations in fieldwork and resource-limited settings, particularly in environments where cold-chain logistics are impractical or unavailable.

In another aspect of the present disclosure, RTV18, RTV18FL, and combinations possess resistance to inhibitors commonly encountered during nucleic acid extraction and those co-purified from biological and clinical samples. RTV18, RTV18FL, and combinations have the ability to maintain activity in the presence of these inhibitors, demonstrating the robustness and reliability in a wide variety of challenging sample types. RTV18, RTV18FL, and combinations have demonstrated a high level of resistance to a broad spectrum of inhibitors, including those encountered during nucleic acid extraction and those present in complex biological and clinical samples. This robustness underscores the suitability of the present disclosure for molecular diagnostics, especially in environments where sample quality may be compromised by contaminants or inhibitors. Compared to conventional reverse transcriptases, such as M-MULV RT, which are highly susceptible to such inhibitors, RTV18, RTV18FL, and combinations represent a major advancement in terms of both resilience and performance under challenging conditions. This capacity to withstand inhibitory substances without significant loss of activity revealed that RTV18, RTV18FL, and combinations are superior enzymes for RT-qPCR and other molecular biology techniques, offering enhanced reliability and robustness in diagnostic and research applications.

An aspect of the present disclosure comprises the reduced risk of RNA secondary structure formation or template degradation, leading to more efficient and reliable cDNA synthesis.

In an embodiment of the present disclosure, the engineering of RTV18FL, through the fusion of RTV18 with the inactive RNase H domain, resulted in a reverse transcriptase with enhanced processivity, capable of efficiently synthesizing full-length cDNA from RNA fragments as long as 9 kb. The modular structure of RTV18FL, which combines the catalytic efficiency of RTV18 with the stabilizing benefits of the inactive RNase H domain, makes it highly suitable for applications requiring the synthesis of long cDNA molecules, such as full-length transcriptome analysis, long-read sequencing, and gene expression profiling. The novel combination of RTV18 with an inactive RNase H domain, resulting in RTV18FL, provides a significant technological advantage, offering enhanced anchoring to RNA templates without the need for active RNA degradation. As a result, RTV18FL can be utilized for a wide range of applications that demand efficient synthesis of longer cDNA fragments, broadening the scope of molecular biology and diagnostic techniques in which it can be applied.

In an embodiment of the present disclosure incorporating 10% RTV18FL into the RTV18 formulation, the overall enzyme mixture benefits from the combined properties of both enzymes, making it an ideal reverse transcriptase for a broad spectrum of molecular biology and diagnostic applications. This formulation is particularly advantageous for protocols requiring fast reaction times without compromising the ability to handle longer RNA templates, such as RNA-seq, long-read cDNA synthesis, and high-temperature reverse transcription assays. Thus, the blending approach offers significant technical advantages over using RTV18 or RTV18FL alone, providing a more versatile and efficient enzyme that meets the demands of a wider range of experimental conditions, particularly in the context of high-temperature and long-template reverse transcription assays. The novel blend formulation thus offers a robust solution for various molecular biology and diagnostic workflows.

An aspect of the present disclosure is that the RTV18, RTV18FL, and combinations are robust for repeated freeze-thaw applications, making it easy to transport and versatile for extended storage. Namely, thermostable reverse transcriptase enzyme wherein the thermostable enzyme is at least 90% identical to at least a sequence selected from a list consisting of: SEQ ID NO: 2, SEQ ID NO: 3, or combinations thereof; preferably 91%, 92%, 93%, 94%, 95%, 96%, 97%, 98%, 99%, identical.

The reverse transcriptase enzyme of the present disclosure exhibits significantly enhanced thermostability, is thermoactivated, shows increased resistance to reverse transcriptase inhibitors, and demonstrates superior efficiency in reverse transcribing challenging templates. Additionally, it offers faster reaction rates, improved specificity, and greater processivity.

In an embodiment, the blended enzyme performs efficiently across a wider range of applications, successfully synthesizing cDNA fragments of 80 bp to 9 kb at temperatures ranging from 30°C to 70°C.

In an embodiment, the incorporation of RTV18FL ensures that longer RNA templates are transcribed efficiently, combining the fast kinetics of RTV18 with the ability to handle long and challenging RNA templates.

In an embodiment, the blend maintains the rapid kinetics of RTV18, allowing cDNA synthesis to be completed in ≤1 minute, even at elevated temperatures.

In an embodiment, the blend retains the high tolerance to inhibitors demonstrated by RTV18, making it suitable for use in complex or inhibitor-rich samples without a loss in performance.

The present disclosure relates to a composition for use in RT-PCR or RT-LAMP polymerase reactions, comprising the enzyme RTV18 and/or RTV18FL. The composition of the present disclosure applies to both diagnostic and research-based applications, improving the efficiency, accuracy, and speed of nucleic acid amplification.

In an embodiment for better results, the sequence encoding the protein of the present disclosure is at least 90% identical to a sequence selected from a list consisting of: SEQ ID NO: 2, SEQ ID NO: 3, or combinations thereof; preferably at least 95%, identical; more preferably at least 98% identical.

In an embodiment for better results, the amino acid sequence encoding the reverse transcriptase RTV18 (SEQ ID NO: 2) of the present disclosure may comprise only one domain, wherein the domain is an N-terminal polymerase catalytic domain.

In an embodiment for better results, the thermostable enzyme of the present disclosure may encode the reverse transcriptase RTV18FL (SEQ ID NO: 3) of the present disclosure, comprising RTV18 (SEQ ID NO: 2) fused with an inactive RNase H domain from Moloney Murine Leukemia Virus reverse transcriptase.

In an embodiment for better results, the thermostable enzyme of the present disclosure, wherein the high catalytic efficiency and high stability may be maintained across a temperature range from 30°C to 50°C; preferably 30°C to 60°C; more preferably 30°C to 70°C.

An aspect of the present disclosure relates to a vector or a construct comprising the thermostable enzyme as described in the present disclosure.

In an embodiment for better results, the vector of the present disclosure may be selected from the group consisting of: virus vector and/or plasmid; preferably a plasmid.

Another aspect of the present disclosure relates to a method for producing the thermostable enzyme of the present disclosure, comprising:
obtaining a vector comprising the thermostable enzyme;
and transforming the said vector in a competent host, wherein the host is a microorganism, preferably *Escherichia coli*;
purifying and obtaining the thermostable enzyme.

Another aspect of the present disclosure relates to a composition for amplifying a nucleic acid sequence, comprising RTV18 (SEQ ID NO: 2) and RTV18FL (SEQ ID NO: 3), and a suitable buffer system for nucleic acid amplification.

In an embodiment for better results, the composition of the present disclosure may comprise:
60-99% (w/w) of RTV18 (SEQ ID NO: 2), preferably 80-95 % (w/w), more preferably 88-92 % (w/w);
1-40% (w/w) of RTV18FL (SEQ ID NO: 3); preferably 5-20 % (w/w), more preferably 6-12 % (w/w).

In an embodiment for better results, the composition of the present disclosure may comprise at least 60%(w/w) RTV18 (SEQ ID NO: 2) and/or at least 40% (w/w) RTV18FL (SEQ ID NO: 3), or at least 70%(w/w) RTV18 (SEQ ID NO: 2) and/or at least 30% (w/w) RTV18FL (SEQ ID NO: 3), or at least 80% (w/w) RTV18 (SEQ ID NO: 2) and/or at least 20%(w/w) RTV18FL (SEQ ID NO: 3), or at least 85%(w/w) RTV18 (SEQ ID NO: 2) and/or at least 15%(w/w) RTV18FL (SEQ ID NO: 3), or at least 95% (w/w) RTV18 (SEQ ID NO: 2) and/or at least 5% (w/w) RTV18FL (SEQ ID NO: 3); preferably at least 90% (w/w) RTV18 (SEQ ID NO: 2) and/or at least 10%(w/w) RTV18FL (SEQ ID NO: 3).

In an embodiment, for better results, the composition of the present disclosure may further comprise a suitable additive selected from: buffer, pH indicator, dye, stabilizer, surfactant, or combinations thereof.

In an embodiment, the composition of the present disclosure may further include a suitable buffer with the following composition: 50 mM Tris-HCl, pH 8.3, 75 mM KCI, 3 mM MgCl2, 10 mM DTT, and 0.25% (v/v) Tween^{®} 20, optimized to enhance the performance of the enzyme.e

An aspect of the present disclosure relates to a method for amplifying a nucleic acid sequence, comprising contacting a nucleic acid template with the composition of the present disclosure under conditions suitable for nucleic acid amplification.

In an embodiment for better results, the nucleic acid amplification may be performed using polymerase chain reaction cycling conditions or using reverse transcription loop-mediated isothermal amplification under isothermal conditions.

In another aspect of the present disclosure, the thermostable enzyme or the composition described in the present disclosure may be used as a reverse transcription improver.

In an embodiment for better results, the thermostable enzyme, or the composition of the present disclosure may be used as an improver of molecular biology techniques.

An aspect of the present disclosure relates to a method of obtaining the thermostable enzyme according to any one of claims 1-5, comprising the steps of:
inoculating a liquid medium with a microorganism containing a gene for the expression of the desired fusion protein;
allowing the microorganism to multiply during a certain incubation period and inducing the expression under the influence of a promoter; and
retrieving the desired fusion protein/peptide from the obtained microorganism by purifying the protein from endogenous contaminants; wherein the gene is synthetically obtained.

### BRIEF DESCRIPTION OF THE DRAWINGS

The following figures provide preferred embodiments for illustrating the disclosure and should not be seen as limiting the scope of the invention.

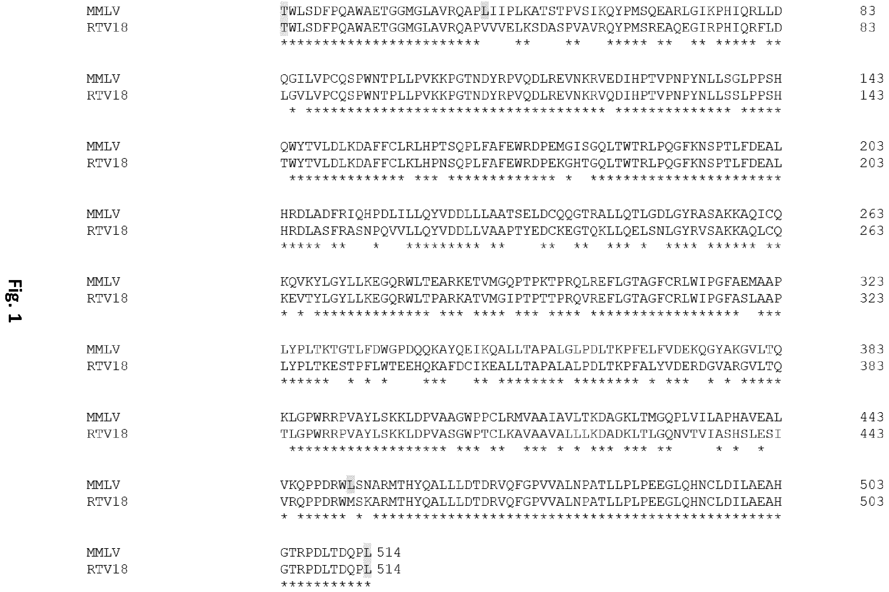

**Figure 1****:** Illustration of results of the primary amino acid sequence alignment of Moloney murine leukemia virus (M-MULV) reverse transcriptase and RTV18. The amino acid sequences of the N-terminal complementary deoxyribonucleic acid (cDNA) polymerase catalytic domain of M-MULV reverse transcriptase (PubMed Protein Accession Number: AAC82568) and RTV18 were aligned. The aligned region spans residues Thr24 to Leu514 (highlighted in blue), corresponding to the truncated version of M-MULV reverse transcriptase used as a reference for the design and engineering of RTV18. The residues Leu48 and Leu452 of M-MULV RT, which flank the region replaced by sequences identified through natural diversity screening to generate chimeric reverse transcriptases (including RTV18), were highlighted in green. These two sequences share 80% identity. Consensus sequences among the aligned proteins are indicated by an asterisk (*).

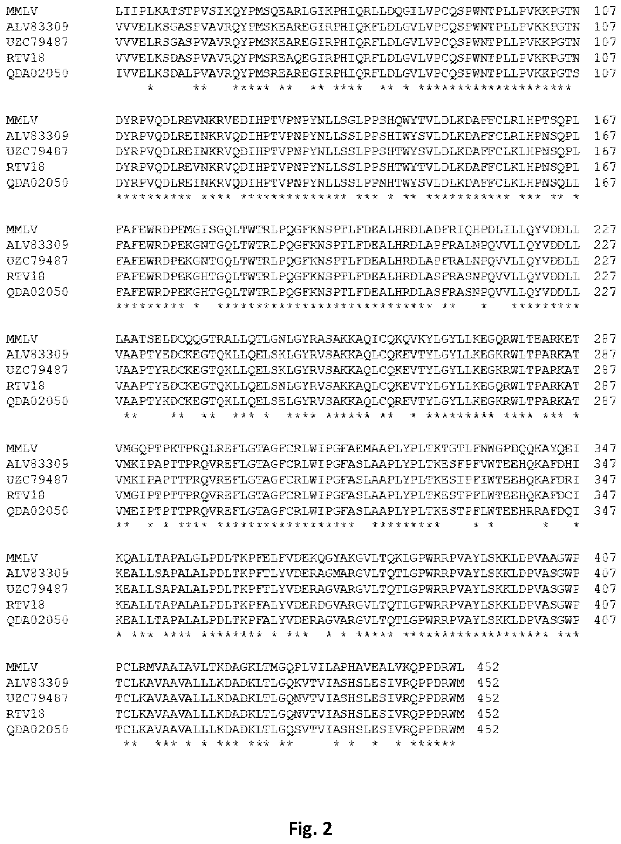

**Figure 2****:** Illustration of results of the sequence alignment of RTV18 and homologous proteins with M-MULV reverse transcriptase. The amino acid sequence of RTV18 was aligned with homologous reverse transcriptases, including Moloney murine leukemia virus (M-MULV) reverse transcriptase. RTV18 shares 76% sequence identity with M-MULV RT across the aligned regions. This alignment serves as the basis for assessing sequence divergence. Consensus sequences among the aligned proteins are indicated by an asterisk (*).

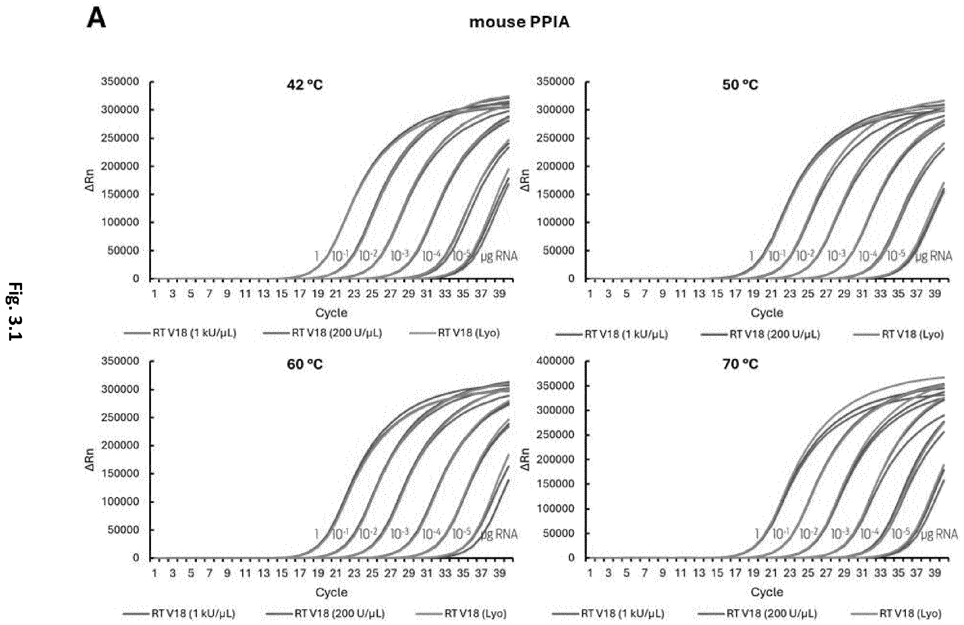

**Figure 3.1** - **3.2****:** Illustration of results of the RTV18 enzymatic activity across a 42°C to 70°C temperature range under three storage conditions. RTV18 was tested in a glycerol-free format at high concentration (1 kU/µlL), in a glycerol format at standard concentration (200 U/µlL), and in lyophilized form (Lyo). A dilution series of mouse total RNA (1 µg to 10 pg) was used as a template for cDNA synthesis performed for 1 minute at 42°C, 50°C, 60°C, and 70°C. Synthesized cDNA was detected using TaqMan primers and probes specific for the *Mus musculus* PPIA (A) and Rpl27 genes (B).

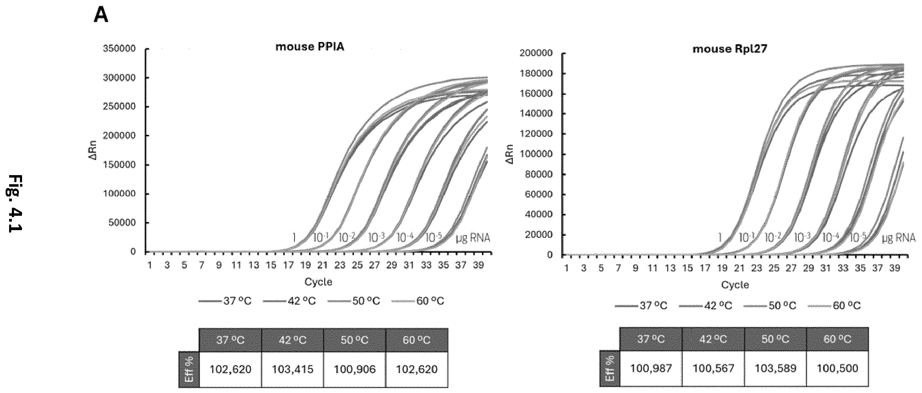

**Figure 4.1** - **4.2****:** Illustration of results of the cDNA synthesis efficiency and Ct values for RTV18 across a temperature range of 37°C to 60°C using the glycerol-preserved enzyme format (200 U/µL). RTV18, at a concentration of 200 U/µL, was used to synthesize cDNA from a dilution series of mouse total RNA (ranging from 1 µg to 10 pg) for 1 minute at four different temperatures: 37°C, 42°C, 50°C, and 60°C. The cDNA synthesis process was monitored using TaqMan primers and probes specific for the *Mus musculus* PPIA and Rpl27 genes. (A) Amplification curves for each target gene were overlaid for the four temperatures, and amplification efficiency (Eff%) was calculated for each condition to assess RTV18's performance across the temperature range. (B) Comparative analysis of Ct values was performed to evaluate the consistency of cDNA synthesis efficiency at each temperature for both gene targets.

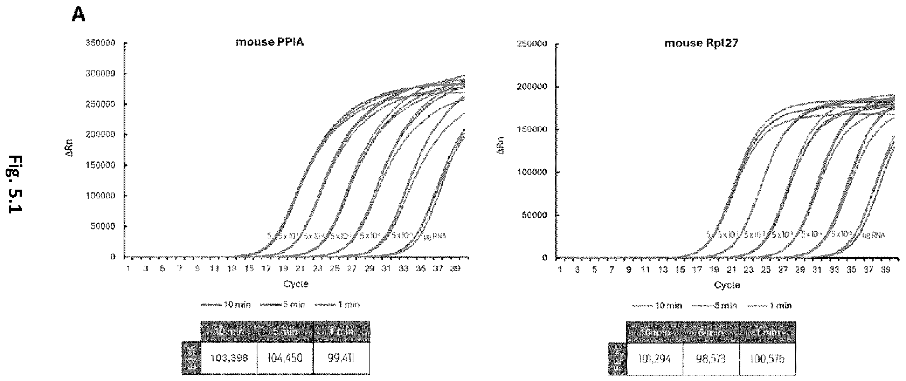

**Figure 5.1** - **5.2****:** Illustration of results of the speed of RTV18 glycerol-preserved enzyme format (200 U/µL) during cDNA synthesis at 60°C. A dilution series of mouse total RNA (5 µg to 50 pg) was used as a template for cDNA synthesis at 60°C for reaction times of 1, 5, and 10 minutes. **(A)** Synthesized cDNA was detected using TaqMan primers and probes specific for the *Mus Musculus* PPIA and Rpl27 genes. **(B)** The Ct values for the amplifications following cDNA synthesis from 5 µg of RNA were compared across the three reaction periods, demonstrating RTV18's capacity for efficient cDNA synthesis at varying time intervals.

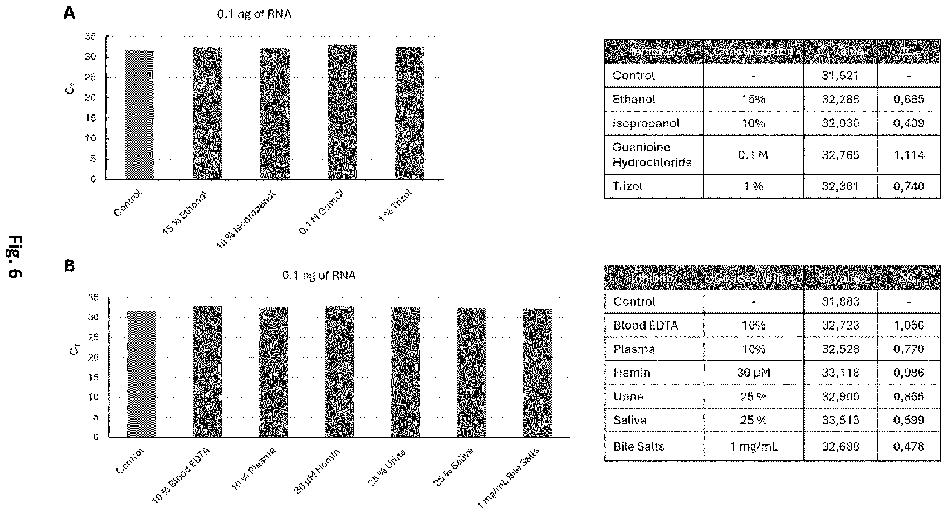

**Figure 6****:** Illustration of results of the RTV18 resistance to inhibitors associated with nucleic acid extraction (A) and commonly co-purified from biological and clinical samples (B). The resistance of RTV18 (glycerol-preserved format, 200 U/µL) was evaluated during cDNA synthesis at 60°C for 1 minute. The reaction used 0.1 ng of total RNA and oligo (dT)₁₈ primers and was conducted with a 1-minute fast protocol at 60°C in the presence of various inhibitors. A control reaction without inhibitors was included. Inhibitor resistance was determined by comparing the difference in Ct values between reactions with and without inhibitors. Synthesized cDNA was quantified through qPCR amplification of the *Mus Musculus* PPIA gene.

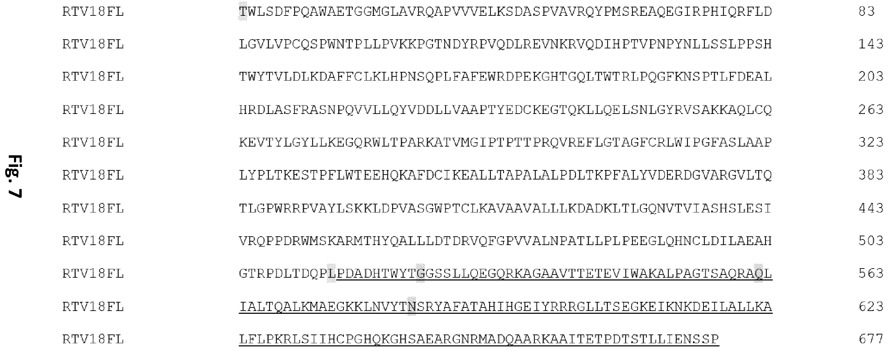

**Figure 7****:** Representation of an embodiment of the present disclosure concerning the primary amino acid sequence of RTV18FL. The primary amino acid sequence of RTV18FL was presented, highlighting key structural features. Residues Thr24 to Leu514 were highlighted in blue, corresponding to the truncated version of RTV18 reverse transcriptase. The C-terminal domain of M-MULV RT, which was fused to RTV18 to create RTV18FL, was underlined. The residues mutated to inactivate the RNase H catalytic function were highlighted in green. This fusion creates a modular enzyme with enhanced processivity compared to RTV18 alone.

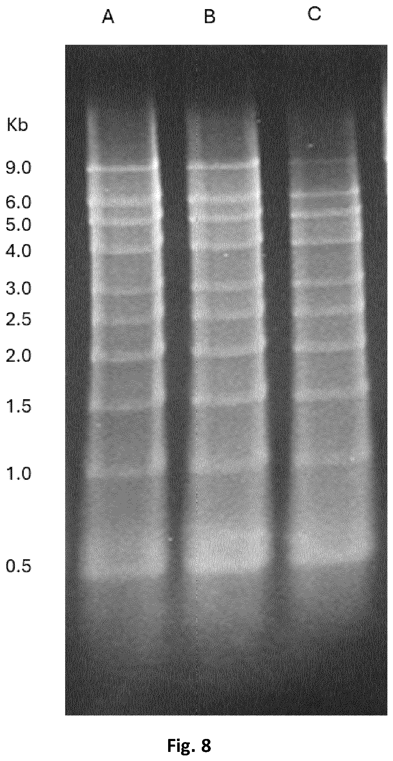

**Figure 8****:** Illustration of results of the processivity of RTV18 fused to the inactive M-MULV RT RNase H module (consisting in RTV18FL). The processivity of RTV18FL was evaluated by its capacity to synthesize full-length cDNA from RNA fragments of varying sizes (0.5 to 9 kb). Reactions with different enzyme concentrations (20, 10, and 5 U/µL, Lanes A to C, respectively) were tested using the enzyme in the glycerol-preserved format, at 200 U/µL stock concentration. Oligo (dT)₁₈ primers were used to reverse transcribe RNA fragments from 1 µg of the Millennium^{™} RNA Marker (Invitrogen^{™}, #AM7150). cDNA synthesis was performed at 50°C for 20 minutes.

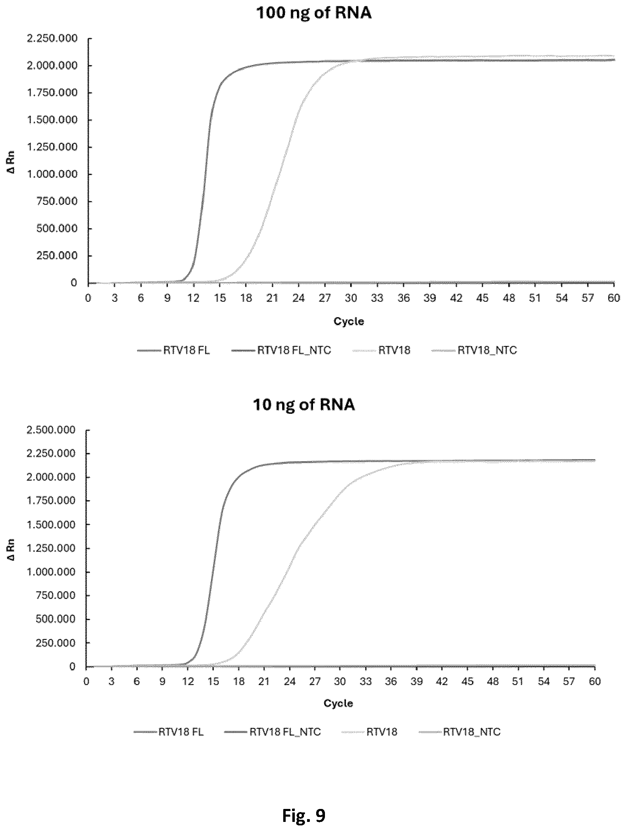

**Figure 9****:** Illustration of results of the efficacy of RTV18 in quantitative reverse transcription loop-mediated isothermal amplification (RT-qLAMP) amplification. The performance of RTV18 fused to the inactive RNase H module of M-MULV RT (designated RTV18FL) was compared to the unfused RTV18 enzyme in RT-LAMP assays. Both enzymes were evaluated based on their capacity to synthesize cDNA from RNA templates at two concentrations (100 ng and 10 ng). The cDNA was subsequently amplified and detected in a real-time qLAMP reaction using Bst DNA Polymerase. The *Mus musculus* B-actin gene was targeted in these assays. Control reactions without template RNA (NTC - No Template Control) were included for both RTV18FL (RTV18FL_NTC) and RTV18 (RTV18_NTC). Efficacy was assessed by comparing the difference in Ct values obtained for each enzyme under the respective conditions.

### DETAILED DESCRIPTION

The present disclosure relates to novel reverse transcriptase enzymes, RTV18, RTV18FL, and combinations offering enhanced performance across a wider temperature range, faster reaction times, and increased resilience to inhibitors, thus improving the stability and the catalytic activity of reverse transcription, enabling a more efficient and reliable molecular biology diagnostic.

The present disclosure relates to newly developed reverse transcriptase enzymes, RTV18, RTV18FL, and their combinations, which demonstrate superior performance over an extended temperature range, faster reaction times, and heightened resistance to inhibitors. These attributes significantly enhance the stability and catalytic efficiency of reverse transcription, leading to more reliable and effective molecular biology applications

### Identification and selection of unknown reverse transcriptases

In the present disclosure, previously uncharacterized RTs were selected from the NCBI non-redundant protein sequence database based on sequence homology to known RTs. These RTs were chosen for their potential to exhibit novel biochemical properties or enhanced enzymatic performance for specific applications. Candidate sequences were identified based on homology to M-MULV reverse transcriptase. The BLASTp analysis (https://blast.ncbi.nlm.nih.gov/Blast.cgi) was conducted using the first 514 residues of Moloney Murine Leukemia Virus (M-MULV) reverse transcriptase (PubMed Protein Accession Number: AAC82568), which corresponds to the N-terminal cDNA polymerase catalytic domain. A gene alignment algorithm was applied to exclude sequences with greater than 90% and less than 70% homology (identity) to M-MULV RT. This selection method ensured that highly similar or highly divergent sequences were excluded, refining the pool to include diverse, yet relevant, candidates. The primary sequences representing this natural diversity were retrieved for further analysis, including multiple sequence alignments and phylogenetic tree generation. These analyses allowed the selection of a subset of RTs that represented a broad range of natural biodiversity, suitable for subsequent biochemical characterization.

### Design and synthesis of gene variants encoding RTs

In an embodiment of the present disclosure, 24 representative unknown reverse transcriptases (RTs) from various species, along with the native Moloney Murine Leukemia Virus (M-MULV) reverse transcriptase (AAC82568), were selected. The corresponding 25 genes were designed by reverse-translating the protein sequences and optimizing codon usage for high expression in Escherichia coli using NZYtech's proprietary ATGenium Codon Optimization Algorithm. The codon optimization was based on the global codon usage patterns in *E. coli,* with particular emphasis on codons frequently used in highly expressed native genes, while avoiding naturally rare codons. The GC content of the designed genes was controlled to range between 40% and 60%, and measures were taken to minimize the presence of G/C-rich islands that could cause frameshifts. Additionally, the sequences were designed to ensure the absence of *E. coli* regulatory elements such as promoters, activators and operators, and the absence of contiguous strings of more than five nucleotides that might interfere with expression. To ensure high expression levels, the Codon Adaptation Index (CAI) was optimized to values above 0.8. The synthetic gene fragments were produced via a high-throughput synthesis pipeline using established and optimized procedures.

### Gene cloning

In an embodiment of the present disclosure, the synthetic gene fragment encoding M-MULV reverse transcriptase was initially cloned into the pHTP1 expression vector (NZYtech) using the NZYEasy Cloning & Expression system (NZYtech, #MB282), generating the plasmid pHTP1mmlv. After the cloning reaction, the recombinant plasmid was transformed into *E. coli* NZY5α competent cells using a high-throughput (HTP) transformation method. The plasmid was recovered from transformed bacterial colonies and fully sequenced in both directions to ensure 100% consistency with the designed gene sequence. The 24 genes encoding the representative unknown reverse transcriptases (RTs) were cloned into pHTP1mmlv by replacing the DNA region corresponding to M-MULV RT residues Leu48 - Leu452. This was achieved using a modified Ligation-Independent Cloning (LIC) method, previously established and optimized for this purpose. Truly, the architecture of the final proteins resulted from the fusion of two different proteins, resulting in the engineering of a library of chimeric proteins with two very short N- and C-terminal regions from M-MULV RT and the bulk large internal part from the unknown enzyme. The entire library of RT variants was sequenced in both directions to verify that no mutations occurred during gene synthesis or cloning. To generate the RTV18FL construct, the DNA sequence encoding the mutated C-terminal domain of M-MULV RT was inserted at the 3'-end of the RTV18 gene using the previously mentioned LIC method. The RNAse H activity was inactivated by introducing mutations at three key catalytic residues: Asp524Gly, Glu562Gln, and Asp583Asn.

### Expression, purification and storage of recombinant RTs

In an embodiment, the 25 pHTP plasmid derivatives were transformed into *E. coli* BL21 (DE3) cells. For protein expression, the 25 recombinant strains were inoculated into 100 mL of NZY auto-induction LB medium (NZYtech, #MB179), supplemented with kanamycin (50 µg/mL). Initial growth was conducted at 37°C until the culture reached an OD600 of 1.5 to 2.0. Following this, the incubation temperature was reduced to 20°C for 16 hours to promote protein expression. After the incubation, OD₆₀₀ was measured to estimate cell density, and the cells were collected by centrifugation. The harvested cells were resuspended and lysed in NZY Bacterial Cell Lysis Buffer (NZYtech, #MB178) supplemented with lysozyme and DNase I. Cell lysis was performed at 25°C for 20 minutes. The cell-free extract was recovered by centrifugation, and the recombinant proteins were purified using Immobilized Metal Affinity Chromatography (IMAC) on an ÄKTA Purifier system (Cytiva). The column was washed twice with Buffer A (50 mM Tris, 300 mM NaCl, 10 mM Imidazole, pH 8.0), and the recombinant proteins were eluted using Buffer A supplemented with 250 mM imidazole. For standard analysis, proteins were stored in protein chromatography elution buffer supplemented with glycerol. The integrity and purity of the recombinant proteins were confirmed through sodium dodecyl sulfate-polyacrylamide gel electrophoresis (SDS-PAGE) using a 14% (w/v) polyacrylamide gel. Protein concentration was measured via the Bradford protein assay (NZYtech, #MB19801), calibrated against a standard curve generated from bovine serum albumin (BSA). The molecular weights and extinction coefficients of the recombinant proteins were calculated using the ExPASy ProtParam tool (http://www.expasy.ch/tools/protparam.html).

In an embodiment, the purified recombinant proteins were stored in three distinct optimized storage buffers, each tailored to preserve enzyme stability under different conditions. These included:
- A glycerol-free buffer optimized for storing the proteins at high concentrations (1 kU/µL),
- A 50% (w/w) glycerol-containing buffer for storage at a concentration of 200 U/µL, and
- A lyophilized format for long-term storage.

Lyophilization of the recombinant reverse transcriptases (RTs) was performed using standard freeze-drying techniques to ensure stability and ease of reconstitution. The lyophilization process was carried out following established protocols. Specific parameters, such as the freezing temperature, drying pressure, and duration of the lyophilization cycle, were optimized to retain protein activity upon rehydration. The resulting lyophilized proteins were stored under controlled conditions to maintain their structural integrity and enzymatic activity over extended periods. These storage conditions were designed to support various downstream applications, ensuring that the recombinant RTs could be reliably used in experimental workflows under a wide range of laboratory conditions.

### cDNA synthesis and quantification

In an embodiment, first-strand cDNA synthesis was performed using serial dilutions of mouse liver total RNA (Takara Bio, #636603) ranging from 5 µg to 10 pg per reaction. The RNA was primed with Oligo (dT)₁₈ primers to target the poly(A) tails of mRNA, allowing for selective cDNA synthesis. To initiate the reaction, 2.5 mM Oligo (dT)₁₈ primer mix (NZYtech, # MB12801), 0.5 mM dNTPs (each dNTP at 10 mM), and the RNA were incubated at 65°C for 5 minutes in a total volume of 13 µL. This incubation step allowed the primers to anneal to the RNA template. Following this, the reaction was rapidly cooled to prevent non-specific annealing. A 7 µL reaction mixture containing 1x Rxn Buffer for NZY Reverse Transcriptase (NZYtech, #MB124), 2 U of NZY Ribonuclease Inhibitor (NZYtech, #MB410) and 20 U of RTV18 reverse transcriptase was added to the primed RNA solution, bringing the final reaction volume to 20 µL. Unless otherwise indicated, the complete reaction mixture was immediately transferred to a thermal cycler for incubation at varying temperatures (37°C, 42°C, 50°C, 60°C, or 70°C) for 1, 5, or 10 minutes. After cDNA synthesis, the reaction was inactivated by heating at 85°C for 5 minutes.

Quantification of the synthesized cDNA was performed using quantitative real-time PCR (qPCR) using the TaqMan probe technology. Each qPCR reaction was set up in a total volume of 20 µL, containing 1x NZYSupreme qPCR Probe Master Mix (NZYtech, #MB416), 4 µM primers, and 1.25 µM TaqMan probes targeting the *Mus musculus* PPIA (IDT Assay ID Hs.PT. 58v.38887593.g), or Rpl27 (SEQ ID NO: 7 - Fw primer-TCCAAGATCAAGTCCTTTGTGA, SEQ ID NO: 8 - Rv primer-GTCCCTGAACACATCCTTGT and probe SEQ ID NO: 9 - ACTACAACCACCTCATGCCCACAA) housekeeping genes. The synthesized cDNA comprised up to 8% (w/w) of the total qPCR reaction volume. Each rection was performed in triplicate. cDNA synthesis efficiency was quantified by plotting Ct values against a standard curve of known RNA concentrations, enabling accurate determination of cDNA synthesis efficiency across a range of RNA input levels. The qPCR amplification was performed using the StepOnePlus^{™} Real-Time PCR System (Applied Biosystems) with the following cycling conditions: 95°C for 2 minutes, followed by 40 cycles of 95°C for 10 seconds and 60°C for 30 seconds.

In an embodiment, to assess the resistance of RTV18 to various PCR inhibitors, related to nucleic acid extraction (15% (w/w) ethanol, 10% (w/w) isopropanol, 0,1 M guanidine hydrochloride and 1% (w/w) trizol) and commonly co-purified from biological and clinical samples (10% (w/w) whole blood EDTA, 10% (w/w) plasma, 30 µM Hemin, 25%(w/w) urine, 25%(w/w) saliva and 1 mg/mL bile salts), the mentioned concentrations of inhibitors were added to the cDNA synthesis reactions. The maximum concentration of each inhibitor was determined in prior experiments (data not shown). The effect of inhibitors on RTV18 activity was quantified by comparing the Ct values from inhibitor-containing reactions with those from control reactions (no inhibitor added). Each reaction was performed in triplicate. The difference in Ct values (ΔCt) was calculated to determine the degree of inhibition.

### Multiplex cDNA synthesis of different size cDNA fragments

In an embodiment, to assess the capacity of RTV18FL to synthesize cDNA from templates of varying sizes, the RNA Millennium^{™} Marker (Invitrogen^{™}, #AM7150) was used as the RNA template. This marker consists of RNA transcripts ranging from 0.5 kb to 9 kb, providing a robust system for evaluating the enzyme's efficiency in multiplex cDNA synthesis across a wide range of transcript lengths. The reaction mixture was prepared following the standard protocol, as described in previous sections, with Oligo (dT)₁₈ primers, dNTPs, and RTV18FL reverse transcriptase in the presence of the Supreme RTase Reaction Buffer (NZYtech). The final reaction volume was 20 µL. To facilitate the synthesis of longer cDNA fragments, the reaction was incubated at 50°C for 20 minutes. This temperature and extended incubation time were optimized to allow for the efficient reverse transcription of larger RNA fragments, ensuring full-length cDNA synthesis even for the longest transcript in the marker set (9 kb).

In an embodiment, following cDNA synthesis, the resulting cDNA products were analysed using agarose gel electrophoresis. The amplified products were separated on a 1.0% (w/v) agarose gel, in TAE buffer, to allow clear resolution of cDNA fragments of different sizes. Post-electrophoresis, the gel was stained with SYBR^{™} Gold Nucleic Acid Gel Stain (1x concentration) for 15 minutes to visualize the synthesized cDNA fragments. SYBR^{™} Gold was chosen for its high sensitivity in detecting nucleic acids, ensuring clear visualization of both small and large cDNA products.

### RT-LAMP assays

In an embodiment, quantitative reverse transcription loop-mediated isothermal amplification (RT-qLAMP) was performed using mouse liver total RNA (Takara Bio, #636603) to amplify the *Mus musculus* β-actin gene. Two template concentrations, 100 ng and 10 ng, were used to assess the enzyme's performance across varying input levels. The RT-qLAMP reaction was carried out in a 25 µL volume, formulated as follows:
- 1x Polaris^{®} BstY Reaction Buffer 10x (NZYtech, #MD0688),
- 6 mM Polaris^{®} MgSO4 (100 mM stock, NZYtech, # MD0689),
- 1x LAMP Primer Mix 10x (comprising 40 µM FIP/BIP, 5 µM F3/B3, 10 µM LoopF/LoopB, in TE Buffer),
- 1.4 µM Polaris^{®} dNTPs mix (25 mM, IVD, NZYtech, # MD0690),
- 0.6x NZY LAMP Fluorescent Dye 50x (NZYtech, #MB45401),
- 0.32 U of Polaris^{®} BstY Polymerase 8 U/µL (NZYtech, # MD06781),
- 6.4 U NZY Ribonuclease Inhibitor (NZYtech, #MB0840), and
- 2 U of the reverse transcriptase under test, either RTV18 Full Length (RTV18FL) or RTV18.

The RT-qLAMP reactions were conducted on the StepOnePlus^{™} Real-Time PCR System (Applied Biosystems) using the following thermal cycling programme, 60 cycles of 69°C for 30 seconds, an inactivation step at 95°C for 3 minutes, followed by a melt curve analysis with temperature increments of 0.5°C at 10-second intervals, covering a range from 65°C to 95°C. The amplification process was monitored in real-time, and fluorescence signals were recorded using the SYBR channel. The melt curve analysis allowed for the differentiation of specific amplified products based on their melting temperatures (Tm), which serves as a verification step to confirm the specificity of the amplified cDNA fragments.

In an embodiment, the RT-qLAMP assay was designed to assess the efficiency of cDNA synthesis and subsequent amplification using two versions of the novel reverse transcriptase, RTV18 and RTV18FL. These enzymes were evaluated for their ability to effectively reverse transcribe RNA templates of varying concentrations and facilitate the amplification process in a rapid, isothermal environment. The use of a LAMP Primer Mix (FIP- SEQ ID NO: 10 AGCGGTGAACCAAGACGCAGGGCGCGATCAAAACAACG, SEQ ID NO: 11 BIP- AATTCCCTCGAGGACAAGGCGAGCTCTTCGGTAGTAGCCAA, SEQ ID NO: 12 F3-CCAGAATGGAGAACGCAGTG, SEQ ID NO: 13 B3- CCGTCACCACCACGAATT, SEQ ID NO: 14 FL- TTATTGGGTAAACCTTGGGGC and SEQ ID NO: 15 BL-TTCCAATTAACACCAATAGCAGTCC) enabled highly specific amplification of the target RNA sequences, while the Polaris^{®} BstY Polymerase (NZYtech, #MD06781) provided strand displacement activity critical for the LAMP process. The presence of NZY LAMP Fluorescent Dye allowed for real-time monitoring of the amplification process, while the addition of NZY Ribonuclease Inhibitor ensured the protection of RNA templates from degradation throughout the reaction. The comparative performance of RTV18 and RTV18FL was evaluated by analyzing the amplification kinetics and the melt curve data. Differences in amplification efficiencies between the two reverse transcriptase variants were quantified by comparing the fluorescence intensity and the melt curve profiles.

### Generation of recombinant RTV18

The gene encoding the N-terminal domain of Moloney Murine Leukemia Virus (M-MULV) reverse transcriptase (see Fig. 1 for the primary sequence) was initially designed and cloned into the pHTP1 expression vector using established molecular cloning techniques. This recombinant plasmid, designated pHTP1mmlv, was transformed into *E*. *coli* BL21 (DE3) for protein expression. The resulting recombinant M-MULV reverse transcriptase was expressed at high levels, predominantly in soluble form, and demonstrated active reverse transcriptase activity, confirming the proper folding and functionality of the expressed protein. Following the successful expression of M-MULV RT, a bioinformatics screening was conducted to identify sequences with less than 90% but greater than 70% identity to the N-terminal cDNA polymerase catalytic domain of M-MULV reverse transcriptase. The screening focused on the first 512 residues of M-MULV reverse transcriptase, which were used as a query to probe the NCBI non-redundant protein sequence database, retrieving approximately 800 candidate reverse transcriptase sequences.

Using multiple sequence alignment and phylogenetic analysis, a subset of 24 representative sequences was selected, covering a broad range of natural diversity. This selection was based on the sequences' evolutionary relationships and their homology to the M-MULV RT N-terminal domain, with the aim of generating chimeric reverse transcriptases for functional testing. DNA fragments corresponding to M-MULV RT residues Leu48 to Leu452 (Fig. 1) of each of the 24 selected genes were designed and synthesized. These fragments were subcloned into the pHTP1mmlv expression vector using a precise DNA assembly technique, replacing the corresponding region of the M-MULV reverse transcriptase, between Leu48 to Leu452, with the selected natural sequences. This generated a library of 24 chimeric reverse transcriptases, each consisting of an N-terminal and a C-terminal region of M-MULV RT and the intervening region from the respective natural RT sequences. All recombinant plasmids were fully sequenced in both directions to ensure that no mutations accumulated during gene synthesis and cloning. The integrity of each plasmid was confirmed before further analysis.

In an embodiment, the library of recombinant reverse transcriptases was expressed in *E. coli* BL21 (DE3) and purified using Immobilized Metal Affinity Chromatography (IMAC). Preliminary biochemical characterization of the chimeric proteins revealed variations in their activity and stability, with RTV18 emerging as a candidate of particular interest due to its enhanced performance. Specifically, RTV18 displayed a broad temperature optimum, allowing efficient activity over a wide range of temperatures (37 to 70°C). Additionally, RTV18 exhibited fast enzyme kinetics, reverse transcribing RNA in just 1 minute, making it a prime candidate for further characterization. RTV18 shares 76% identity with M-MULV RT reverse transcriptase, with four gaps in the alignment of the two sequences (as shown in Fig. 2). This sequence alignment, alongside similar natural sequences to RTV18, highlights areas of both convergence and divergence, providing insights into the structural and functional adaptations of RTV18. Based on its superior biochemical properties, RTV18 was selected for detailed biochemical characterization, which is described in subsequent sections. The primary sequence of the RTV18 chimeric protein characterized below is fully presented in Fig. 1.

### RTV18 storage conditions and stability analysis

In an embodiment, to ensure the versatility and stability of RTV18 for diverse applications, the enzyme was stored under three distinct conditions, optimized for different experimental and practical uses:
- Glycerol-free high-concentration buffer: RTV18 was stored in a glycerol-free buffer optimized for high-concentration storage, with protein concentrations reaching 1 kU/µL. This formulation, 0,1 M Trehalose, 0,2 M Sorbitol, 0,3 M Betaine, 2 mM DTT, 0,1 mM EDTA e 0,15% (w/w) Tween 20, is particularly suited for applications where high enzyme concentrations are required, such as high-throughput assays or large-scale enzymatic reactions. The absence of glycerol in this buffer is beneficial for downstream processes like lyophilization, where glycerol might interfere with protein preparation or activity.
- 50%(w/w) Glycerol-containing buffer: RTV18 was also stored in a 50% glycerol-containing buffer, which preserves the enzyme at a concentration of 200 U/µL. The inclusion of glycerol, a well-known cryoprotectant, is intended to protect the enzyme from freeze-thaw damage, and this format is ideal for routine laboratory use, where flexibility in freezing and thawing cycles is critical.
- Lyophilized format: For long-term storage and transportation, RTV18 was lyophilized following standard freeze-drying protocols. Lyophilization ensures that RTV18 can be stored in a stable, dehydrated form, with rapid rehydration and full activity recovery upon use. This format is particularly advantageous for field applications or settings where cold-chain logistics may be challenging.

In an embodiment, the stability of RTV18 across these storage conditions was rigorously tested under various stress conditions, including repeated freeze-thaw cycles and storage at ambient temperatures. The following stability parameters were evaluated:
- Freeze-Thaw Stability: RTV18, stored under all three conditions (glycerol-free, glycerol-containing, and lyophilized formats), was subjected to 10 freeze-thaw cycles, simulating typical laboratory conditions where enzymes are frequently frozen and thawed for repeated use. At the conclusion of the 10 cycles, RTV18's enzymatic activity was assessed using a standard cDNA synthesis assay, with more than 98% of the original activity consistently recovered across all storage formats. This demonstrates RTV18's strong resilience to freeze-thaw damage, particularly in the glycerol-containing and lyophilized formats, making it a robust enzyme for repeated freeze-thaw applications.
- Room Temperature Stability: RTV18's stability was further evaluated by storing the enzyme at room temperature (22°C) for a period of two weeks. Enzymatic activity was assessed at the end of the incubation period, and results confirmed that RTV18 maintained over 98% of its original activity in all storage formats, including the glycerol-free, glycerol-containing, and lyophilized formats. This indicates that RTV18 is suitable for applications where refrigeration or freezing may not be possible, such as fieldwork or use in resource-limited settings.
- Stability of Reconstituted Lyophilized Format: The lyophilized RTV18 was reconstituted and subjected to the same stability tests described above, including freeze-thaw cycles and room temperature incubation. After the stability experiments, the enzyme retained over 98% of its original activity, demonstrating the robustness and stability of RTV18 even after lyophilization and reconstitution. This confirms the lyophilized format's effectiveness for long-term storage and subsequent use, offering a versatile solution for extended storage and ease of transport.

In an embodiment, the availability of RTV18 in multiple storage formats significantly enhances its applicability across a wide range of experimental conditions and settings. The glycerol-free, high-concentration buffer format allows for efficient enzyme use in high-throughput or large-scale reactions, where enzyme concentration is a limiting factor. The 50% glycerol format offers flexibility for routine lab use, particularly where frequent freezing and thawing are required, without compromising enzyme activity. Finally, the lyophilized format provides a robust solution for long-term storage and transport, particularly in field applications or environments where maintaining a cold chain is impractical. By maintaining stability and activity across all storage conditions, RTV18 proves to be a versatile and reliable enzyme for molecular biology applications, from basic research to large-scale industrial processes.

### RTV18 Activity Across a Broad Temperature Range

In an embodiment, the enzymatic activity of RTV18 was evaluated in its three storage formats-glycerol-free, glycerol-containing, and lyophilized-across a temperature range from 42°C to 70°C. This study aimed to assess the enzyme's stability and performance under varying conditions, especially in comparison to Moloney Murine Leukemia Virus (M-MULV) reverse transcriptase, which exhibits optimal activity between 42°C and 50°C but shows limited functionality outside this range.

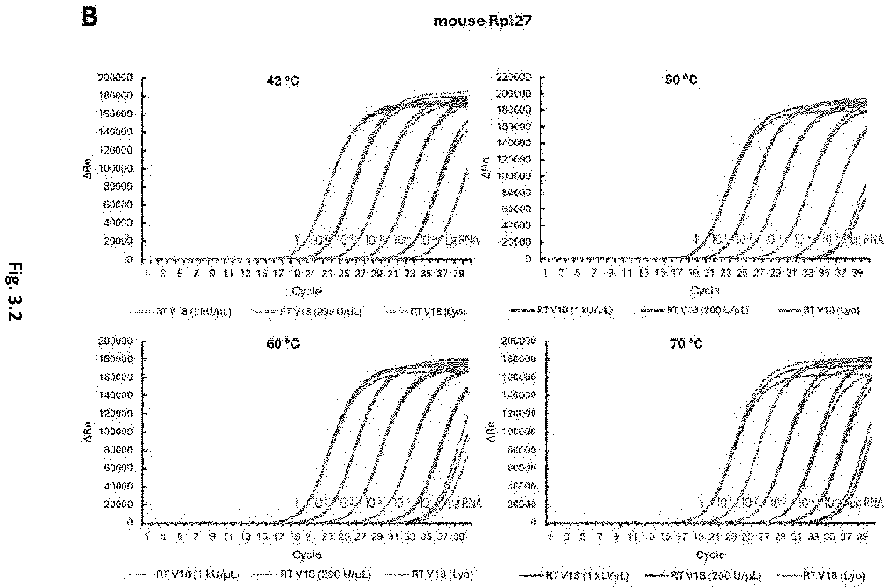

In an embodiment, mouse liver RNA was used as the template for cDNA synthesis, with RNA input quantities ranging from 1 µg to 10 pg per reaction. The cDNA synthesis efficiency of RTV18 was quantified via quantitative PCR (qPCR) targeting two housekeeping genes: PPIA and Rpl27. Results, presented in Fig. 3, demonstrate that RTV18 maintains robust enzymatic activity across the full temperature range, regardless of its storage format. Comparable performance in synthesizing cDNA was observed across all three formats, confirming RTV18's stability and activity at temperatures well beyond M-MULV's restricted range of 42°C to 50°C, which is often used as the benchmark in molecular biology applications.

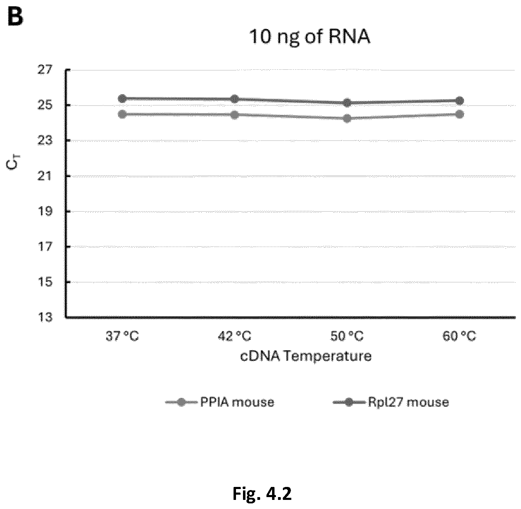

In an embodiment, to further exemplify RTV18's efficiency and robustness, the enzyme's activity was assessed between 37°C and 60°C using the glycerol-preserved RTV18 (200 U/µL). In this experiment, a dilution series of mouse total RNA, ranging from 1 µg to 10 pg per reaction, was used to synthesize cDNA. Quantitative PCR was performed to evaluate the efficiency of the reaction using TaqMan primers and probes specific to PPIA and Rpl27. The results, as illustrated in Fig. 4, revealed that RTV18 maintains near-perfect efficiency across the entire temperature range, with amplification efficiencies consistently near 100%. Even at low RNA input levels (10 pg), RTV18 showed high sensitivity and reproducibility across different temperatures, with consistent Ct values for both target genes.

In an embodiment, RTV18's ability to function efficiently at temperatures as high as 70°C represents a significant improvement over M-MULV reverse transcriptase, which loses activity above 50°C. The broad temperature range (37°C to 70°C) exhibited by RTV18 makes it a versatile enzyme for a variety of molecular biology techniques, including those requiring higher reaction temperatures, such as quantitative reverse transcription polymerase chain reaction (RT-qPCR), RT-LAMP, and other cDNA synthesis applications where thermostability is advantageous. Key findings from the temperature activity study include:
- Broad Temperature Optimum: RTV18 displayed consistent activity across the entire temperature range, from 37°C to 70°C, while M-MULV RT is known to exhibit optimal activity only between 42°C and 50°C. This broader temperature range gives RTV18 a distinct advantage in applications that require flexibility in reaction conditions.
- Equivalent Performance Across Storage Formats: RTV18 exhibited similar activity in all three storage formats - glycerol-free, glycerol-containing, and lyophilized - highlighting the enzyme's stability in each condition. This confirms that RTV18's enzymatic function is preserved across various storage methods, ensuring flexibility for different laboratory and field settings without compromising activity.
- Consistency Across RNA Input Levels: RTV18 maintained consistent performance regardless of the RNA input level, with minimal variation in Ct values even at the lowest input concentration of 10 pg of RNA. This stability across a wide range of RNA concentrations further underscores RTV18's utility in applications that require low RNA input, such as single-cell transcriptomics or degraded RNA samples.
- Superior Temperature Stability Compared to M-MULV RT: M-MULV reverse transcriptase, widely used in molecular biology, is limited to a narrow temperature range for optimal activity. In contrast, RTV18 demonstrates exceptional flexibility, maintaining high performance even at 60°C, where M-MULV RT becomes largely inactive. This makes RTV18 an ideal tool for molecular biology and diagnostic applications requiring high-temperature reactions, where secondary structure formation in RNA is minimized.
- Sensitivity at Low RNA Levels and High Temperatures: RTV18's ability to synthesize cDNA efficiently from as little as 10 pg of RNA at temperatures as high as 60°C illustrates the enzyme's robustness. At the lowest RNA input level, minimal variation in Ct values was observed across the temperature range, confirming RTV18's ability to handle challenging templates, such as those with low RNA concentrations, while maintaining high efficiency.

In an embodiment, the experimental data firmly establish RTV18 as a highly efficient reverse transcriptase with a broad temperature range, significantly superior to the more temperature-restricted M-MULV RT enzyme. RTV18's capacity to maintain near-100% efficiency across this wide temperature spectrum and various RNA input levels underscores its potential as a powerful tool for applications that require both high sensitivity and temperature flexibility. Its ability to function efficiently at temperatures as high as 70°C makes RTV18 particularly valuable for protocols requiring elevated temperatures, where the risk of RNA secondary structure formation or template degradation is minimized, leading to more efficient and reliable cDNA synthesis.

### RTV18 velocity in cDNA synthesis

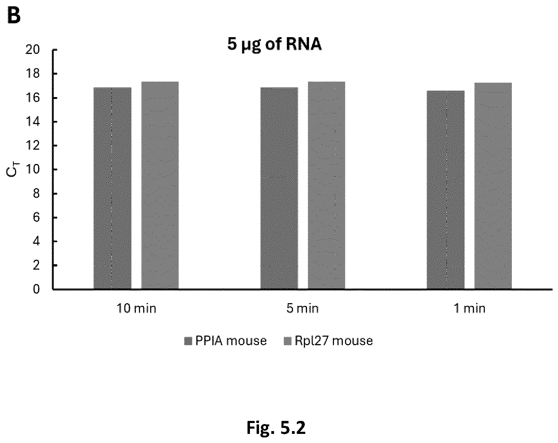

In an embodiment, the velocity of RTV18 was assessed based on its ability to synthesize cDNA at 60°C within reaction periods of 1, 5, and 10 minutes. The objective of this study was to determine RTV18's kinetic performance. It is well known that of the well-characterized M-MULV reverse transcriptase, typically requires 30 minutes to synthesize cDNA from 1 µg of RNA at 42°C. A dilution series of mouse total RNA (ranging from 5 µg to 50 pg) was used as the template for cDNA synthesis. RTV18, preserved in its glycerol format (200 U/µL), was tested at 60°C for reaction times of 1, 5, and 10 minutes. Synthesized cDNA was quantified using quantitative PCR (qPCR) with TaqMan primers and probes specific to two housekeeping genes, PPIA and Rpl27. The results, presented in Fig. 5, demonstrate that RTV18 maintains high efficiency and velocity in cDNA synthesis, irrespective of the reaction time. Key findings from the velocity study are:
- High Velocity at 1-Minute Reaction Time: Even at the shortest reaction period of 1 minute, RTV18 exhibited high efficiency in synthesizing cDNA, as shown by the consistent Ct values for both PPIA and Rpl27. This rapid performance contrasts sharply with M-MULV RT, which typically requires 30 minutes for a comparable reaction. RTV18's ability to produce substantial amounts of cDNA in just 1 minute at 60°C underscores its accelerated kinetics.
- Comparable Kinetics Across 1, 5, and 10-Minute Reaction Times: Remarkably, RTV18 demonstrated similar cDNA synthesis kinetics for 1, 5, and 10-minute reactions, as indicated by the near-identical Ct values across the different reaction times. This suggests that the enzyme reaches maximal cDNA synthesis efficiency within the first minute of the reaction, making it a highly efficient tool for rapid cDNA generation in applications where time efficiency is critical.
- Sensitivity Across a Broad RNA Input Range: The enzyme maintained consistent performance across a wide range of RNA input levels, from 5 µg to 50 pg, irrespective of the reaction time. This further confirms RTV18's utility in applications requiring low RNA input concentrations, such as single-cell RNA sequencing or samples with degraded RNA.
- Superior Performance Compared to M-MULV RT: In contrast to M-MULV RT, which requires significantly longer reaction times at 42°C, RTV18 achieves comparable or better performance in just a fraction of the time at 60°C. This makes RTV18 a far more efficient enzyme for molecular biology and diagnostic applications, particularly in protocols that demand rapid cDNA synthesis.

In an aspect of the present disclosure, RTV18's ability to efficiently synthesize cDNA in as little as 1 minute at 60°C represents a major improvement over traditional reverse transcriptases such as M-MULV RT, which require much longer reaction times and lower reaction temperatures. The rapid kinetics of RTV18 make it a highly versatile tool for various applications where fast reaction times are essential, such as RT-qPCR, RT-LAMP, and other time-sensitive molecular biology protocols. Furthermore, the enzyme's ability to maintain high efficiency and sensitivity across a broad range of RNA inputs and reaction times reinforces its superiority as a next-generation reverse transcriptase for research and diagnostics.

### Resistance of RTV18 to PCR inhibitors

In an aspect of the present disclosure, the resistance of RTV18 to inhibitors commonly encountered during nucleic acid extraction and those co-purified from biological and clinical samples was evaluated under conditions that closely mimic real-world molecular biology and diagnostic workflows. The enzyme's ability to maintain activity in the presence of these inhibitors demonstrates its robustness and reliability in a wide variety of challenging sample types.

In an embodiment, RTV18 (glycerol-preserved format, 200 U/µL) was used for cDNA synthesis from 0.1 ng of total RNA at 60°C for 1 minute. The primers used in the reaction were oligo (dT)₁₈, which anneal to the poly(A) tail of mRNA. Various inhibitors known to be associated with nucleic acid extraction and those present in biological or clinical samples were added to the reaction to evaluate their impact on the enzyme's performance. A control reaction without inhibitors was included for comparative analysis. Quantitative PCR (qPCR) was subsequently used to amplify the *Mus Musculus* PPIA gene, and the difference in Ct values (ΔCt) between the control reaction and the inhibitor reactions was calculated to determine inhibitor resistance.

In an embodiment, the results, presented in Fig. 6, illustrate that RTV18 exhibits remarkable resistance to a wide range of inhibitors. Inhibitors commonly associated with nucleic acid extraction, including ethanol (15%), isopropanol (10%), guanidine hydrochloride (0.1 M), and trizol (1%), were evaluated for their impact on RTV18's activity. The data in Fig. 6A reveal that RTV18 maintains high enzymatic activity in the presence of all these inhibitors, with only minor shifts in Ct values compared to the control reaction without inhibitors. The minor increase in Ct values across these conditions confirms that RTV18 can effectively resist inhibition, maintaining activity even in the presence of high concentrations of inhibitors typically encountered during the RNA extraction process.

In an embodiment, RTV18's performance was also tested against inhibitors commonly found in biological and clinical samples, including blood EDTA (10%), plasma (10%), hemin (30 µM), urine (25%), saliva (25%), and bile salts (1 mg/mL). The results in Fig. 6B show that RTV18 maintained high levels of activity even in the presence of these inhibitors, with minimal changes in Ct values. The minor Ct shifts across these inhibitors underscore RTV18's potential for use in clinical diagnostics, where sample purity cannot always be guaranteed. RTV18's ability to resist inhibition from common clinical contaminants makes it particularly valuable for point-of-care diagnostics and other settings where rapid and reliable nucleic acid amplification is critical.

In an aspect of the present disclosure, RTV18 has demonstrated a high level of resistance to a broad spectrum of inhibitors, including those encountered during nucleic acid extraction and those present in complex biological and clinical samples. This robustness underscores RTV18's suitability for molecular diagnostics, especially in environments where sample quality may be compromised by contaminants or inhibitors. Compared to conventional reverse transcriptases, such as M-MULV RT, which are highly susceptible to such inhibitors, RTV18 represents a major advancement in terms of both resilience and performance under challenging conditions. This capacity to withstand inhibitory substances without significant loss of activity positions RTV18 as a superior enzyme for real-time PCR and other molecular biology techniques, offering enhanced reliability and robustness in diagnostic and research applications.

### RTV18 Processivity through C-terminal Fusion with Inactive RNase H Domain

Processivity, a key characteristic of reverse transcriptases, refers to an enzyme's ability to synthesize long cDNA molecules from RNA templates without prematurely dissociating.

In an embodiment, fusing RTV18 with an inactive C-terminal RNase H domain of M-MULV reverse transcriptase provided the necessary structural anchorage for more efficient engagement with RNA, particularly for longer RNA molecules. The inactive RNase H domain, while not contributing to the enzyme's catalytic activity, has been shown to play a role in enhancing the processivity of reverse transcriptases by stabilizing interactions with the RNA template, allowing for the synthesis of longer cDNA products.

In an embodiment, a novel derivative of RTV18, termed RTV18FL, incorporated the inactive C-terminal RNase H domain of M-MULV RT (see Fig. 7 for the primary sequence of RTV18FL). This modification was aimed at evaluating whether this modular enzyme could outperform the original RTV18 in synthesizing long cDNA fragments. The processivity of RTV18FL was evaluated by its capacity to synthesize full-length cDNA from RNA fragments of varying sizes, ranging from 0.5 kb to 9 kb. Millennium^{™} RNA Marker (Invitrogen^{™}, #AM7150) was used as the template, containing RNA fragments of the following sizes: 0.5, 1, 1.5, 2, 2.5, 3, 4, 5, 6, and 9 kb. For each reaction, 1 µg of the RNA marker was used, and the reverse transcription reaction was conducted at 50°C for 20 minutes.

In an embodiment, the RTV18FL enzyme, preserved in glycerol format at a stock concentration of 200 U/µL, was tested at varying concentrations - 20, 10, and 5 U/µL (as shown in Lanes A to C, respectively in Fig. 8). Oligo (dT)₁₈ primers were utilized to prime the poly(A) tails of the RNA templates, ensuring uniform initiation of cDNA synthesis. The synthesized cDNA fragments were then analysed by gel electrophoresis to visualize the enzyme's capacity to transcribe long RNA fragments. The results, illustrated in Fig. 7, clearly demonstrate that RTV18FL exhibited a significant capacity to generate long cDNA products. RTV18FL efficiently synthesized full-length cDNA from RNA templates up to 9 kb. The comparison of cDNA products across the different enzyme concentrations revealed that even at the lower enzyme concentrations of 10 U/µL and 5 U/µL, RTV18FL maintained its ability to synthesize longer cDNA fragments.

In an embodiment, this enhanced processivity of RTV18FL is attributed to the structural contribution of the inactive RNase H domain. This domain likely acts as a stabilizing scaffold, improving RTV18FL's interaction with the RNA template throughout the reverse transcription process. The RNase H domain does not participate in catalysis but enhances the enzyme's anchoring capacity to the RNA, thus allowing for the synthesis of longer cDNA molecules. At an enzyme concentration of 20 U/µL, RTV18FL was able to transcribe all RNA fragments ranging from 0.5 kb to 9 kb, indicating that the enzyme's processivity had been significantly enhanced through the fusion of the RNase H domain.

In an embodiment, the engineering of RTV18FL, through the fusion of RTV18 with the inactive RNase H domain, resulted in a reverse transcriptase with enhanced processivity, capable of efficiently synthesizing full-length cDNA from RNA fragments as long as 9 kb. The modular structure of RTV18FL, which combines the catalytic efficiency of RTV18 with the stabilizing benefits of the inactive RNase H domain, makes it highly suitable for applications requiring the synthesis of long cDNA molecules, such as full-length transcriptome analysis, long-read sequencing, and gene expression profiling. The novel combination of RTV18 with an inactive RNase H domain provides a significant technological advantage, offering enhanced anchoring to RNA templates without the need for active RNA degradation. As a result, RTV18FL can be utilized for a wide range of applications that demand efficient synthesis of longer cDNA fragments, broadening the scope of molecular biology and diagnostic techniques in which it can be applied.

### RTV18FL Efficacy in RT-LAMP Assays

RT-LAMP (Reverse Transcription Loop-Mediated Isothermal Amplification) is a robust technique for the rapid and sensitive detection of RNA targets, leveraging isothermal conditions for efficient amplification.

In an embodiment, the RTV18FL enzyme, a fusion of RTV18 with the inactive RNase H domain of M-MULV RT, was evaluated for its performance in RT-LAMP assays compared to the original RTV18 enzyme. This comparison aimed to assess whether the fusion of the inactive RNase H module enhances RTV18's efficiency in reverse transcription, particularly in the context of LAMP, a critical tool in molecular diagnostics. The efficacy of RTV18FL and RTV18 was tested using mouse liver total RNA (Takara Bio, #636603) to amplify the *Mus musculus* β-actin gene at two different concentrations, 100 ng and 10 ng. cDNA synthesis was followed by a real-time LAMP reaction using Polaris^{®} BstY Polymerase 8 U/µL (NZYtech, #MD0678) to amplify and detect the synthesized cDNA. The performance of each enzyme was assessed by comparing their Ct values, which represent the cycle threshold where the amplified signal becomes detectable. Control reactions (NTC) without the RNA template were included for both enzymes to verify specificity and rule out non-specific amplification. As shown in Fig. 9, RTV18FL consistently outperformed RTV18 in RT-LAMP assays. At both RNA concentrations, RTV18FL demonstrated lower Ct values, indicating more efficient cDNA synthesis and subsequent amplification. The fusion of the inactive RNase H domain appears to enhance RTV18's capacity to bind and transcribe RNA templates, improving overall processivity. This improvement was especially notable at the lower RNA concentration (10 ng), where the ability to retain the RNA template during reverse transcription is more critical. Both RTV18 and RTV18FL enzymes showed no amplification in the NTC controls, confirming the specificity of the reactions.

In an embodiment, the performance of RTV18FL in RT-LAMP assays, as indicated by the lower Ct values, underscores its superiority for applications requiring strand displacement activity. The incorporation of the inactive RNase H module into RTV18 has substantially improved its performance in RT-LAMP assays. While the RNase H domain is known for its role in degrading RNA during reverse transcription, the inactive module may still contribute to stabilizing the enzyme-template interaction. In RT-LAMP, the RNA template undergoes complex looping and primer binding interactions. The inactive RNase H domain might provide structural stabilization or improved anchoring of the enzyme to the RNA template. Given that LAMP assays are typically conducted at temperatures between 65°C and 70°C, an RT with enhanced functionality at these elevated temperatures, such as RTV18FL, also offers a distinct advantage for RT-LAMP reactions.

### Blending of RTV18 and RTV18FL for Enhanced Enzymatic Performance

In an embodiment, a novel combination of these two reverse transcriptases (RTV18 and RTV18FL) exhibited superior properties. Specifically, a blend comprising 90% RTV18 and 10% RTV18FL was found to be particularly effective; however, other blend ratios, with RTV18FL comprising between 1% and 40%, have also demonstrated comparable efficacy. This combination integrates the advantageous characteristics of both enzymes into a single preparation. The blending of RTV18, known for its rapid kinetics, broad temperature stability, and intrinsic resistance to inhibitors, with RTV18FL, which exhibits enhanced processivity and efficiency in synthesizing long RNA templates, results in an enzyme formulation with unique and synergistic properties. This mixture retains the fast kinetics and broad temperature tolerance of RTV18 while benefiting from the improved processivity conferred by the inactive RNase H domain present in RTV18FL.

In an embodiment, when testing cDNA synthesis for qPCR applications, the blend of RTV18 and RTV18FL showed similar efficacy in synthesizing small cDNA fragments (<200 bp) as both RTV18 and RTV18FL alone, without compromising sensitivity. The blend performed equally well in singleplex and multiplex assays, demonstrating its versatility. Additionally, in multiplex one-step qPCR reactions, the 90:10 blend showed no significant reduction in sensitivity compared to RTV18 alone.

In an aspect of the present disclosure incorporating 10% RTV18FL into the RTV18 formulation, the overall enzyme mixture benefits from the combined properties of both enzymes, making it an ideal reverse transcriptase for a broad spectrum of molecular biology and diagnostic applications. This formulation is particularly advantageous for protocols requiring fast reaction times without compromising the ability to handle longer RNA templates, such as RNA-seq, long-read cDNA synthesis, and high-temperature reverse transcription assays. Thus, the blending approach offers significant technical advantages over using RTV18 or RTV18FL alone, providing a more versatile and efficient enzyme that meets the demands of a wider range of experimental conditions, particularly in the context of high-temperature and long-template reverse transcription assays. The novel blend formulation thus offers a robust solution for various molecular biology and diagnostic workflows.

In an embodiment, the reverse transcriptase sequences can be selected from a list consisting of:

| **SEQ ID NO** | **Seq. name** | **Organism name** | **Molecule type** | **Qualifier Molecule Type** | **Residues** |
|---|---|---|---|---|---|
| **SEQ ID NO: 1** | M-MULV | *Moloney Murine Leukemia Virus* | AA | protein | MGSSHHHHHHSSGLVPRGSHMASTWLSDFPQAWAETGGMGLAVRQAPLIIPLKATSTPVSIKQYPMSQ EARLGIKPHIQRLLDQGILVPCQSPWNTPLLPVKKPGTNDYRPVQDLREVNKRVEDIHPTVPNPYNLLSGL PPSHQWYTVLDLKDAFFCLRLHPTSQPLFAFEWRDPEMGISGQLTWTRLPQGFKNSPTLFDEALHRDLAD FRIQHPDLILLQYVDDLLLAATSELDCQQGTRALLQTLGNLGYRASAKKAQICQKQVKYLGYLLKEGQRWL TEARKETVMGQPTPKTPRQLREFLGTAGFCRLWIPGFAEMAAPLYPLTKTGTLFNWGPDQQKAYQEIKQ ALLTAPALGLPDLTKPFELFVDEKQGYAKGVLTQKLGPWRRPVAYLSKKLDPVAAGWPPCLRMVAAIAVL TKDAGKLTMGQPLVILAPHAVEALVKQPPDRWLSNARMTHYQALLLDTDRVQFGPVVALNPATLLPLPE EGLQHNCLDILAEAHGTRPDLTDQPLLEHHHHHH |
| **SEQ ID NO: 2** | RTV18 | synthetic construct | AA | protein | MGSSHHHHHHSSGLVPRGSHMASTWLSDFPQAWAETGGMGLAVRQAPVVVELKSDASPVAVRQYP MSREAQEGIRPHIQRFLDLGVLVPCQSPWNTPLLPVKKPGTNDYRPVQDLREVNKRVQDIHPTVPNPYNL LSSLPPSHTWYTVLDLKDAFFCLKLHPNSQPLFAFEWRDPEKGHTGQLTWTRLPQGFKNSPTLFDEALHR DLASFRASNPQVVLLQYVDDLLVAAPTYEDCKEGTQKLLQELSNLGYRVSAKKAQLCQKEVTYLGYLLKEG QRWLTPARKATVMGIPTPTTPRQVREFLGTAGFCRLWIPGFASLAAPLYPLTKESTPFLWTEEHQKAFDCI KEALLTAPALALPDLTKPFALYVDERDGVARGVLTQTLGPWRRPVAYLSKKLDPVASGWPTCLKAVAAVA LLLKDADKLTLGQNVTVIASHSLESIVRQPPDRWMSKARMTHYQALLLDTDRVQFGPVVALNPATLLPLPE EGLQHNCLDILAEAHGTRPDLTDQPLLEHHHHHH |
| **SEQ ID NO: 3** | RTV18FL | synthetic construct | AA | protein | MGSSHHHHHHSSGPQQGLRTLNIEDEHRLHETSKEPDVSLGSTWLSDFPQAWAETGGMGLAVRQAPV VVELKSDASPVAVRQYPMSREAQEGIRPHIQRFLDLGVLVPCQSPWNTPLLPVKKPGTNDYRPVQDLREV |
| | | | | | NKRVQDIHPTVPNPYNLLSSLPPSHTWYTVLDLKDAFFCLKLHPNSQPLFAFEWRDPEKGHTGQLTWTRL PQGFKNSPTLFDEALHRDLASFRASNPQVVLLQYVDDLLVAAPTYEDCKEGTQKLLQELSNLGYRVSAKKA QLCQKEVTYLGYLLKEGQRWLTPARKATVMGIPTPTTPRQVREFLGTAGFCRLWIPGFASLAAPLYPLTKE STPFLWTEEHQKAFDCIKEALLTAPALALPDLTKPFALYVDERDGVARGVLTQTLGPWRRPVAYLSKKLDP VASGWPTCLKAVAAVALLLKDADKLTLGQNVTVIASHSLESIVRQPPDRWMSKARMTHYQALLLDTDRV QFGPVVALNPATLLPLPEEGLQHNCLDILAEAHGTRPDLTDQPLPDADHTWYTGGSSLLQEGQRKAGAA VTTETEVIWAKALPAGTSAQRAQLIALTQALKMAEGKKLNVYTNSRYAFATAHIHGEIYRRRGLLTSEGKEI KNKDEILALLKALFLPKRLSIIHCPGHQKGHSAEARGNRMADQAARKAAITETPDTSTLLIENSSP |
| **SEQ ID NO: 4** | mPpia Forward primer | synthetic construct | DNA | other DNA | CAAACACAAACGGTTCCCAG |
| **SEQ ID NO: 5** | mPpia Reverse primer | synthetic construct | DNA | other DNA | TTCACCTTCCCAAAGACCAC |
| **SEQ ID NO: 6** | mPpia Probe | synthetic construct | DNA | other DNA | TGCTTGCCATCCAGCCATTCAG |
| **SEQ ID NO: 7** | Rpl27 Forward primer | synthetic construct | DNA | other DNA | TCCAAGATCAAGTCCTTTGTGA |
| **SEQ ID NO: 8** | Rpl27 Reverse primer | synthetic construct | DNA | other DNA | GTCCCTGAACACATCCTTGT |
| **SEQ ID NO: 9** | Rpl27 Probe | synthetic construct | DNA | other DNA | ACTACAACCACCTCATGCCCACAA |
| **SEQ ID NO: 10** | LAMP Primer Mix FIP | synthetic construct | DNA | other DNA | AGCGGTGAACCAAGACGCAGGGCGCGATCAAAACAACG |
| **SEQ ID NO: 11** | LAMP Primer Mix BIP | synthetic construct | DNA | other DNA | AATTCCCTCGAGGACAAGGCGAGCTCTTCGGTAGTAGCCAA |
| **SEQ ID NO: 12** | LAMP Primer Mix F3 | synthetic construct | DNA | other DNA | CCAGAATGGAGAACGCAGTG |
| **SEQ ID NO: 13** | LAMP Primer Mix B3 | synthetic construct | DNA | other DNA | CCGTCACCACCACGAATT |
| **SEQ ID NO: 14** | LAMP Primer Mix FL | synthetic construct | DNA | other DNA | TTATTGGGTAAACCTTGGGGC |
| **SEQ ID NO: 15** | LAMP Primer Mix BL | synthetic construct | DNA | other DNA | TTCCAATTAACACCAATAGCAGTCC |

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (over the whole the sequence) alignment of two sequences, maximizing the number of matches and minimizing the number of gaps. The BLAST algorithm (Altschul et al.

(1990) J Mol Biol 215: 403-10) calculates sequence identity percentages and performs a statistical analysis of the similarity between sequences. The software for performing BLAST analysis is publicly available through the National Centre for Biotechnology Information (NCBI). Global percentages of similarity and identity may also be determined using one of the methods available in the MatGAT software package (Campanella et al., BMC Bioinformatics. 2003 Jul 10; 4:29. MatGAT: an application that generates similarity/identity matrices using protein or DNA sequences). Minor manual editing may be performed to optimise alignment between conserved motifs, as would be understood by a person skilled in the art. The sequence identity values, which are indicated in the present subject matter as a percentage were determined over the entire amino acid sequence using BLAST with default parameters.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following dependent claims further set out particular embodiments of the disclosure.

## Claims

1. Thermostable reverse transcriptase enzyme,
wherein the thermostable enzyme is at least 90% identical to at least a sequence selected from a list consisting of: SEQ ID NO: 2, SEQ ID NO: 3, or combinations thereof.

2. Thermostable enzyme according to the previous claim, wherein the sequence encoding the protein is at least 90% identical to a sequence selected from a list consisting of: SEQ ID NO: 2, SEQ ID NO: 3, or combinations thereof; preferably at least 95%, identical; more preferably at least 98% identical.

3. Thermostable enzyme according to any of the previous claims, wherein SEQ ID NO: 2 comprises only one domain, wherein the domain is an N-terminal polymerase catalytic domain.

4. Thermostable enzyme according to any of the previous claims, comprising SEQ ID NO: 2 fused with an inactive RNase H domain from Moloney murine leukemia virus reverse transcriptase.

5. Thermostable enzyme according to claims 1-4, wherein the high catalytic efficiency and high stability is maintained across a temperature range from 30°C to 50°C; preferably 30°C to 60°C; more preferably 30°C to 70°C.

6. A vector or a construct comprising the thermostable enzyme as described in any of the previous claims; preferably wherein the vector is selected from the group consisting of: virus vector and/or plasmid; preferably a plasmid

7. Method for producing the thermostable enzyme according to the previous claims 1-4, comprising:
obtaining a vector comprising the thermostable enzyme;
transforming the said vector in a competent host, wherein the host is a microorganism, preferably *Escherichia coli*;
purifying and obtaining the thermostable enzyme.

8. Composition for amplifying a nucleic acid sequence, comprising SEQ ID NO: 2 and SEQ ID NO: 3, and a suitable buffer system for nucleic acid amplification.

9. Composition according to any of the previous claims, comprising:
60-99% (w/w) of SEQ ID NO: 2, preferably 80-95 % (w/w), more preferably 88-92 % (w/w);
1-40% (w/w) of SEQ ID NO: 3; preferably 5-20 % (w/w), more preferably 6-12 % (w/w).

10. Composition according to any of the previous claims, comprising at least 60% (w/w) SEQ ID NO: 2and/or at least 40% (w/w) SEQ ID NO: 3, or at least 70% (w/w) SEQ ID NO: 2and/or at least 30% (w/w) SEQ ID NO: 3, or at least 80% (w/w) SEQ ID NO: 2and/or at least 20% (w/w) SEQ ID NO: 3, or at least 85% (w/w) SEQ ID NO: 2and/or at least 15% (w/w) SEQ ID NO: 3, or at least 95% (w/w) SEQ ID NO: 2 and/or at least 5% (w/w) SEQ ID NO: 3; preferably at least 90% (w/w) SEQ ID NO: 2 and/or at least 10% (w/w) SEQ ID NO: 3.

11. Composition according to any of the previous claims, the composition of the present disclosure may further comprise a suitable additive selected from: buffer, pH indicator, dye, stabilizer, surfactant, or combinations thereof.

12. Composition according to any of the previous claims, wherein the suitable buffer; comprise: Tris-HCl, KCl, MgCl2, DTT, and Tween^{®} 20; preferably 50 mM Tris-HCl, pH 8.3, 75 mM KCI, 3 mM MgCl2, 10 mM DTT, and 0.25% (v/v) Tween^{®} 20.

13. Method for amplifying a nucleic acid sequence, comprising contacting a nucleic acid template with the thermostable enzyme according to any of the previous claims 1-5 under conditions suitable for nucleic acid amplification; preferably the nucleic acid amplification is performed using polymerase chain reaction cycling conditions or using reverse transcription loop-mediated isothermal amplification under isothermal conditions.

14. Use of the thermostable enzyme according to any of the claims 1-5, or the composition according to any of the claims 9-13 as an improver of molecular biology techniques; and/or as a reverse transcription improver.

15. A method of obtaining the thermostable enzyme according to any one of claims 1-5, comprising the steps of:
inoculating a liquid medium with a microorganism containing a gene for the expression of the desired fusion protein;
allowing the microorganism to multiply during a certain incubation period and inducing the expression under the influence of a promoter; and
retrieving the desired fusion protein/peptide from the obtained microorganism by purifying the protein from endogenous contaminants; wherein the gene is synthetically obtained.
